# EUROPÄISCHE PATENTSCHRIFT

(11) **EP 1 974 700 B1**
(45) Veröffentlichungstag und Bekanntmachung des Hinweises auf die Patenterteilung: **22.05.2013**
(21) Anmeldenummer: 08004044.7
(22) Anmeldetag: 05.03.2008
(51) Int. Cl.: A61F 2/90

(54) **Stent mit radial expandierbarem Grundkörper**
Stent with radially expandable body
Stent avec un corps de base radialement extensible

(30) Priorität: 31.03.2007 DE 102007015670
(43) Veröffentlichungstag der Anmeldung: 01.10.2008
(73) Patentinhaber: Biotronik VI Patent AG, 6341 Baar (CH)
(72) Erfinder: Lootz, Daniel, 18119 Rostock (DE); Surber, Bettina, 8600 Dübendorf (CH); Wintsch, Daniel, 8052 Zürich (CH); Riedmüller, Johannes, 90411 Nürnberg (DE)
(74) Vertreter: Lindner-Vogt, Karin L.

(56) Entgegenhaltungen:
- WO-A-2004/105642
- WO-A-2006/029617
- WO-A-2006/087621
- WO-A-2007/117960
- US-A- 5 741 327
- US-A1- 2002 188 347
- US-A1- 2004 236 406
- US-A1- 2006 004 437
- US-A1- 2006 069 424

## Beschreibung

Die Erfindung betrifft einen Stent mit den im Oberbegriff des Patentanspruches angegebenen Merkmalen.

Ein derartiges Gefäßimplantat ist in der WO 2004/103 215 A1 ausführlich beschrieben, insbesondere im Hinblick auf die grundsätzlichen Anwendungsgebiete solcher Stents und deren besondere Problematik etwa beim Einsatz von Materialien mit einer geringen Bruchdehnung und auch geringeren Festigkeiten, wie beispielsweise Magnesiumlegierungen.

Bei der dieser Druckschrift entnehmbaren Ausführungsform ist der Stent durch einen eine Zylinderform umschreibende, aus einer kontrahierten Ausgangsstellung in eine dilatierte Stützstellung radial expandierbaren Grundkörper realisiert, der einerseits eine Vielzahl in Umfangsrichtung umlaufende, in axialer Richtung aneinandergereihte Tragsegmente umfasst. Diese sind jeweils durch eine in ihrer kontrahierten Ausgangsstellung in Grobstruktur mäanderförmige Strebe mit alternierend gegenläufigen Mäanderbögen aus biegsamem Material gebildet. Andererseits weist der Grundkörper in axialparalleler Richtung verlaufende Axialverbinder auf, die die Tragsegmente zwischen Zenitpunkten zumindest eines Teils der Mäanderbögen verbinden.

In der Druckschrift WO 2006/029617 A1 ist eine Stützprothese für Gefäße oder intrakorporale Lumina bechrieben, die eine Vielzahl von Stützringen aufweist, die in eine Längsrichtung mit Hilfe von nichtmetallischen Verbindungselementen verbunden sind.

Die Druckschrift US 5,741,327 offenbart radioopake Marker-Elemente, die an den Enden eines radial expandierbaren Stents angebracht sind.

Die oben erwähnten Magnesiumlegierungen als Material zur Herstellung von Stents besitzen erheblich geringere Festigkeitswerte, als übliche Konstruktionswerkstoffe für ballonexpandierbare Stents, wie beispielsweise medizinischer Stahl mit den Werkstoffbezeichnungen 316L, MP35N oder L605. Diese geringeren Festigkeitswerte bringen in der praktischen Anwendung der Stents Probleme mit sich. So ist es für die Stentplatzierung erforderlich, den Stent auf einem Ballonkatheter zu montieren. Dazu wird der aus einem Hülsenmaterial beispielsweise durch Laserschneiden strukturierte Stent in seine kontrahierte Ausgangsstellung auf den Ballonkatheter gecrimpt. In diesem Zustand wird beim Implantieren der Stent auf dem Kathetersystem auch durch gekrümmte Bereiche des Einführ- oder Blutgefäßsystems geführt. Bei den entsprechenden Passagen können sich einzelne Streben des Stents öffnen, wodurch die Haltekräfte des Stents auf dem Katheter reduziert werden. Dies bedingt ein erhebliches Risiko für einen Verlust des Stents.

Besonders gefährdete Bereiche des Stents sind die endständigen Strebenelemente mit ihren Mäanderbögen. Durch abstehende Randsegmente - das so genannte "Flaring" - besteht zusätzlich die Gefahr, dass neben der mechanischen Reizung der inneren Gefäßwand beim Passieren der Stenose oder dem Zurückziehen in den Einführkatheter ein unbeabsichtigtes Abstreifen des Stents vom Ballonkatheter erfolgt.

Ausgehend von der geschilderten Problematik liegt der Erfindung die Aufgabe zugrunde, einen Stent der gattungsgemäßen Art so zu verbessern, dass ein unbeabsichtigtes Aufweiten des Stents insgesamt oder exponierter Bereiche, wie der endständigen Stirnkanten, während des Implantierens zuverlässig verhindert wird.

Diese Aufgabe wird erfindungsgemäß durch die im Kennzeichnungsteil des Anspruches 1 angegebenen Merkmale gelöst, wonach Mittel zur Stabilisierung der Streben-Grobstruktur in ihrer kontrahierten Ausgangsstellung gegen ein radiales Aufweiten in das Stent-Design integriert sind. Diese Stabilisierungsmittel sind dann bei der eigentlichen radialen Expansion des Stents selbsttätig lösbar.

Mit Vorteil sichern diese Stabilisierungsmittel eine radiale Arretierung des Stents in seinem gecrimpten Zustand, sodass aufgrund dieser Fixierung ein unbeabsichtigtes Dehnen des Stents auch in Teilbereichen vermieden wird.

Gemäß bevorzugten Ausführungsformen der Erfindung können diese Stabilisierungsmittel auf unterschiedliche Weise realisiert werden, wie beispielsweise durch auftrennbare

Verklebungen zwischen benachbart liegenden Mäanderbögen einer Strebe oder von Strebe zu Strebe, durch Rastelemente in der Stent-Struktur, randseitige Fixiertraversen oder Feinstrukturverstrebungen gegen das erwähnte "Flaring". Nähere Erläuterungen hierzu sind der nachfolgenden Beschreibung entnehmbar, in der Ausführungsbeispiele des Erfindungsgegenstandes anhand der beigefügten Zeichnungen näher erläutert werden. Es zeigen:
- Fig. 1: eine perspektivische Darstellung eines Stents in seiner kontrahierten Ausgangsstellung mit einer endständigen Verklebung als Stabilisierungsmittel,
- Fig. 2 und 3: Detaildraufsichten auf den Stent in abgewickelter Darstellung mit zwei unterschiedlichen Verklebungsvarianten,
- Fig. 4: einen schematischen Detailausschnitt in abgewickelter Darstellung eines Stents mit Verrastungen als Stabilisierungsmittel,
- Fig. 5 und 6: schematische Detailausschnitte in abgewickelter Darstellung von Stents mit randseitigen Fixiertraversen,
- Fig. 7: eine schematische Detailansicht in abgewickelter Darstellung eines Stents mit randseitigen Feinstrukturverstrebungen als Stabilisierungsmittel,
- Fig. 8: eine vergrößerten Detailausschnitt aus Fig. 7,
- Fig. 9: eine perspektivische Teildarstellung eines Stents mit Verlängerungsstreben zur lösbaren Befestigung an einem Ballonkatheter,
- Fig. 10: eine Draufsicht auf den Stent gemäß Fig. 9 in abgewickelter Darstellung,
- Fig. 11: eine Detaildraufsicht auf einen Stent in abgewickelter Darstellung mit aneinander gekoppelten Markerfortsätzen, und
- Fig. 12: eine Detaildraufsicht eines Stents in abgewickelter Darstellung mit ringgekoppelten Markerfortsätzen.

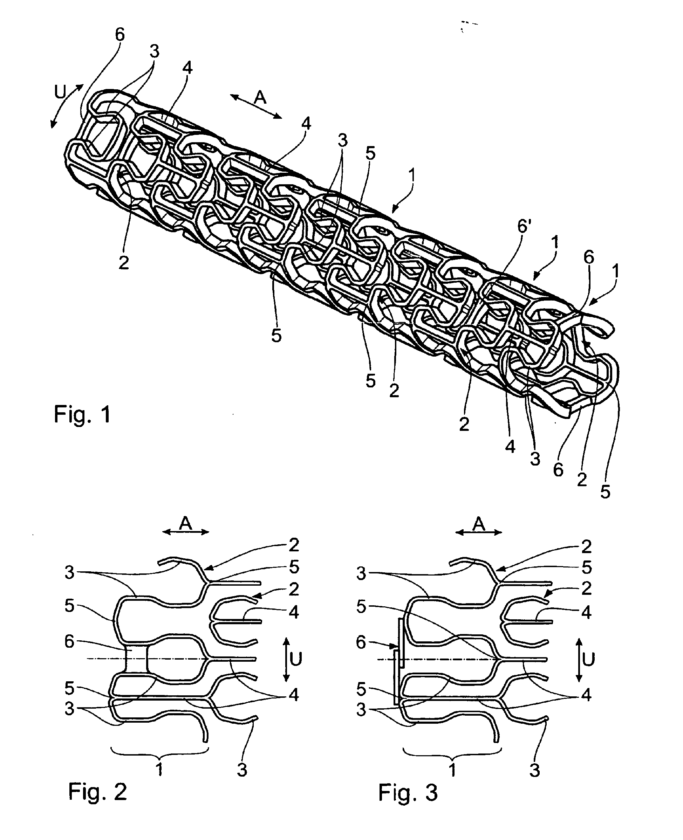

Die Fig. 1 macht die Struktur eines Stents in seiner kontrahierten Ausgangsstellung klar. Der Stent ist aus einem zylindrischen Metallkörper durch Laserschneiden so herausgearbeitet, dass er eine Vielzahl in Umfangsrichtung U umlaufender, in Axialrichtung A aneinandergereihter Tragsegmente 1 aufweist, die jeweils durch eine in der gezeigten kontrahierten Ausgangsstellung in ihrer Grobstruktur mäanderförmige Strebe 2 gebildet sind.

In zur Axialrichtung A paralleler Richtung sind die Tragsegmente 1 durch Axialverbinder 4 miteinander verbunden, die jeweils zwischen den Zenitpunkten 5 jeweils zu verbindender Mäanderbögen 3 verlaufen. Durch den Versatz der benachbarten Tragsegmente 1 in Umfangsrichtung U verlaufen die Axialverbinder 4 immer von der Außenseite eines Mäanderbogens 3 zur Innenseite des Mäanderbogens 3 der benachbarten Strebe 2.

Wie in den Zeichnungen nicht näher dargestellt wird, werden beim radialen Expandieren des Stents die zwischen den Zenitpunkten 5 verlaufenden Abschnitte der Mäanderbögen 3 in Umfangsrichtung U aufgestellt. Je näher sich die entsprechenden Abschnitte dabei an die Umfangsrichtung U annähern, desto größer ist der so genannte Kollapsdruck des Stents.

Um die gemäß der Erfindungsaufgabe erwünschte zusätzliche radiale Fixierung des Stents zu erreichen, ist bei der in Fig. 1 bis 3 gezeigten Variante eine Verklebung 6 vorgesehen, die beispielsweise zwischen den beiden endständigen Mäanderbögen 3 an diametral gegenüberliegenden Radialpositionen in Form eines Vergusspunktes abgebracht sein kann. In Fig. 2 ist diese Verklebung 6 schematisch dargestellt. Bei dieser Art von Verklebung 6 werden die Klebeflächen auf Zug belastet.

Alternativ dazu ist es auch möglich, die Verklebung 6 so anzubringen, dass die Klebeflächen auf Scherung belastet werden, wie dies Fig. 3 andeutet.

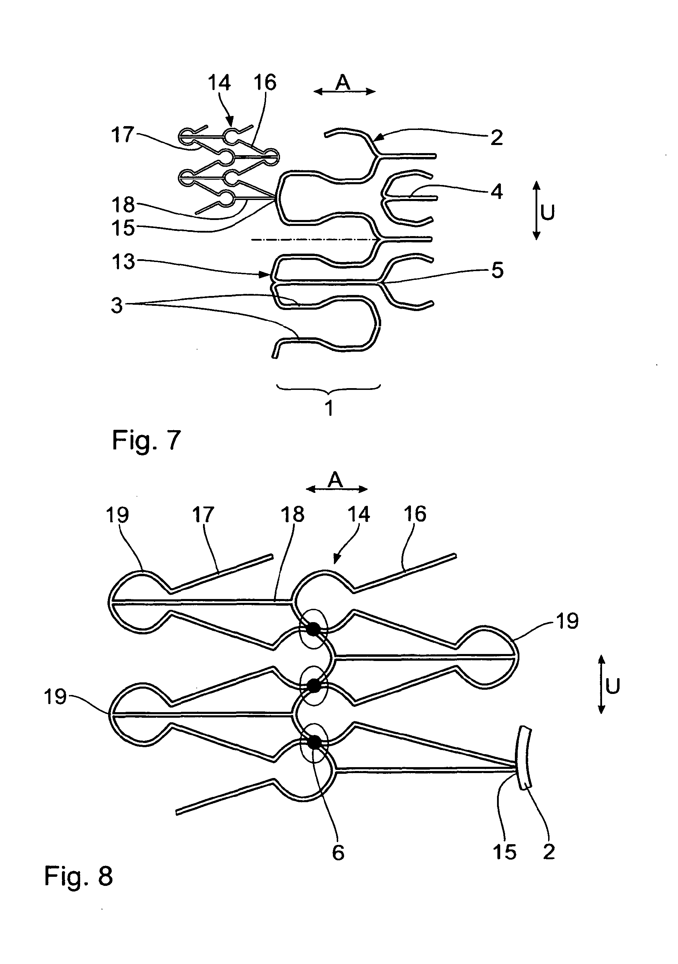

Die einzelnen Punkte der Verklebung 6 können nicht nur endständig, wie in Fig. 1 bis 3 gezeigt, sondern auch über die Länge des Stents 1 verteilt am Umfang angeordnet sein. Bei einer solchen Mehrfachanordnung wird beim radialen Expandieren des Stents die Fixierung entsprechend der Haltekraft in zeitlicher Aufeinanderfolge selbsttätig gelöst. Versuche haben dabei gezeigt, dass trotz einer asymmetrischen Lösung der einzelnen Fixierungen sich der Stent homogen öffnen lässt, sofern die Fixierkraft nur so groß ist, dass die Axialverbinder 4 diese noch aufzunehmen vermögen. Bei einer Anordnung beispielsweise von Verklebungen 6' im Bereich der jeweils zweitäußersten Strebe 2 können die Axialverbinder 4 im Übrigen von beiden Seiten auf die Verklebung 6' einwirken, was einem homogenen Öffnungsverhalten zugute kommt. In diesem Falle sollte die äußerste Strebe 2 entweder möglichst kurz sein oder die im Folgenden anhand der Fig. 7 und 8 noch näher beschriebenen Feinstrukturen am Rand aufweisen, um ein Flaring zu unterdrücken.

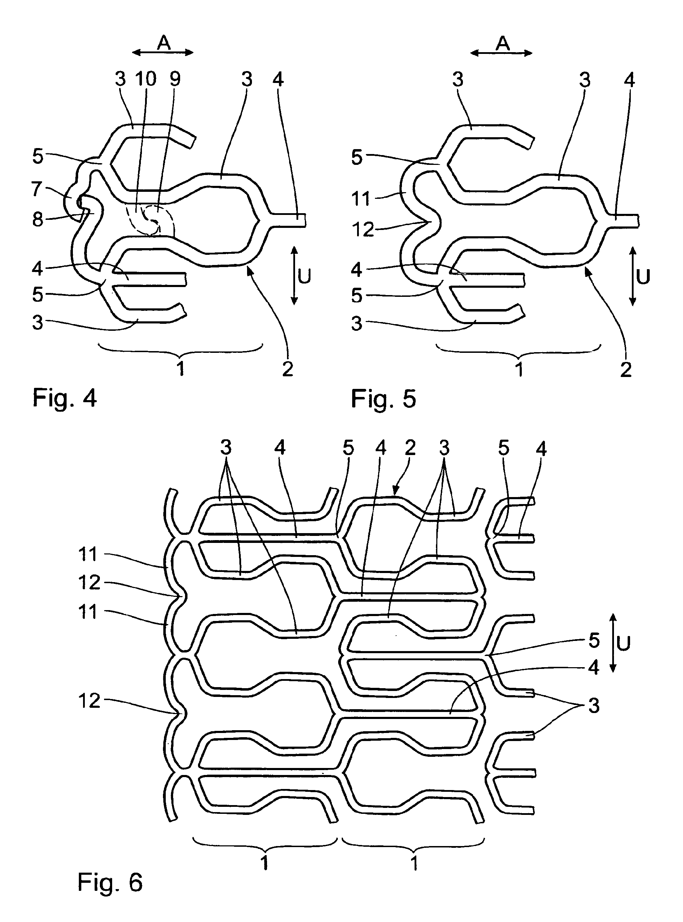

Fig. 4 zeigt eine alternative Möglichkeit für das Stabilisierungsmittel zur zusätzlichen radialen Fixierung des Stents. Dort sind zwei benachbarte Mäanderbögen 3 einer Strebe 2 randseitig durch hakenförmige Rastelemente 7, 8 in der kontrahierten Ausgangsstellung miteinander gekoppelt. Dies erfolgt, indem beim Überführen des Stents in seine kontrahierte Ausgangsstellung - dem so genannten Crimpen - die Rastelemente 7, 8 miteinander in Eingriff gebracht werden. Somit ist der Stent an seinem Rand zusammengehalten, sodass dem eingangs erwähnten "Flaring" vorgebeugt wird. Beim radialen Aufweiten wird die Verrastung zwischen den Elementen 7, 8 aufgebrochen und der Stent kann sich homogen öffnen.

In Fig. 4 ist ferner strichliert eine alternative Anordnung von Rastelementen in Form von Hakenvorsprüngen 9, 10 im Inneren eines Mäanderbogens 3 dargestellt. Auch diese Fixierung wird beim Crimpen durch Einhaken der Vorsprünge 9, 10 hergestellt und beim Expandieren des Stents aufgebrochen. Es ist erkennbar, dass die Hakenvorsprünge 9, 10 an beliebigen Streben 2 entlang des Stents vorgesehen werden können.

Die Fig. 5 und 6 zeigen weitere Varianten für die Stabilisierungsmittel zur zusätzlichen radialen Fixierung des Stents im gecrimpten Zustand. In dieser Ausführungsform sind einstückig an die randseitigen Streben 2 angeformte Fixiertraversen 11 vorgesehen, die zwei benachbarte Mäanderbögen 3 einer Strebe 2 von Zenitpunkt 5 zu Zenitpunkt 5 verbinden. Während in Fig. 5 lediglich eine Fixiertraverse 11 vorgesehen ist, ist bei der Ausführungsform gemäß Fig. 6 der Rand des Stents komplett mit einer Reihe von Fixiertraversen 11 abgeschlossen.

Jede Fixiertraverse 11 weist einen doppelbogenförmigen Verlauf mit einem zentralen Knick 12 auf, der als Sollbruchstelle beim radialen Expandieren des Stents wirkt. Durch das Auftrennen der Fixiertraversen 11 kann sich der Stent - wie bereits im Zusammenhang mit der Verklebung 6 geschildert - homogen aufweiten.

Eine weitere Ausführungsform für die erfindungsgemäß vorgesehenen Stabilisierungsmittel ist in den Fig. 7 und 8 gezeigt. Es handelt sich dabei um eine randseitig an den Enden 13 des Stents umlaufende Feinstrukturverstrebung 14, die an die Außenseiten 15 der Mäanderbögen 3 angebunden ist. Diese Feinstrukturverstrebung 14 ist jeweils aus zwei im Wesentlichen parallel verlaufenden Streben 16, 17 mit in Axialrichtung verlaufenden Koppelstreben 18 gebildet. Die Grundkonfiguration der Feinstrukturverstrebung 14 ist also leiterartig, wobei die Doppelstreben 16, 17 in der Mantelebene des Stents zick-zack-förmig gefaltet sind. In den Knickpunkten sind dabei bogenförmige Zwickelpunkte 19 in die Doppelstreben 16, 17 eingeformt, über die die Koppelstreben 18 materialschonend hinsichtlich des radialen Aufweitens der Feinstrukturverstrebung 14 an den Streben 16, 17 einstückig befestigt sind. Durch die Bogenform in diesen Zwickelpunkten 19 wird die Aufdehnbarkeit der Feinstrukturverstrebung 14 verbessert.

Wie in Fig. 8 schematisch angedeutet ist, ist die Feinstrukturverstrebung 14 im Bereich der in der kontrahierten Ausgangsstellung einander benachbarten, inneren Zwickelpunkte 19 aneinander beispielsweise durch eine Verklebung 6 oder auch hier nicht dargestellte Rastmittel zusätzlich fixiert. Damit ist der gesamte Stent an seinem stirnseitigen Rand in der vercrimpten Konfiguration so stabilisiert, dass ein Flaring möglichst gering gehalten wird. Durch die Fixierung bei den innen liegenden Zwickelpunkten 19 wirken die Öffnungskräfte beidseitig dieser Fixierung, sodass höhere Fixierkräfte überwunden werden können, ohne die Stentstruktur unzulässig zu verziehen.

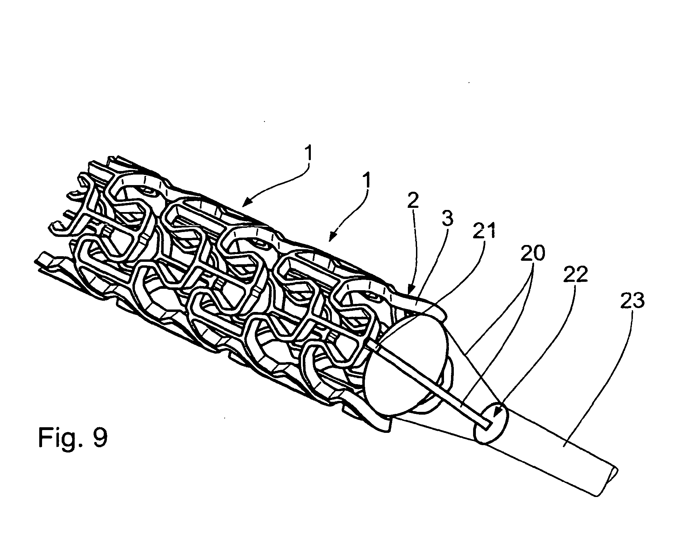

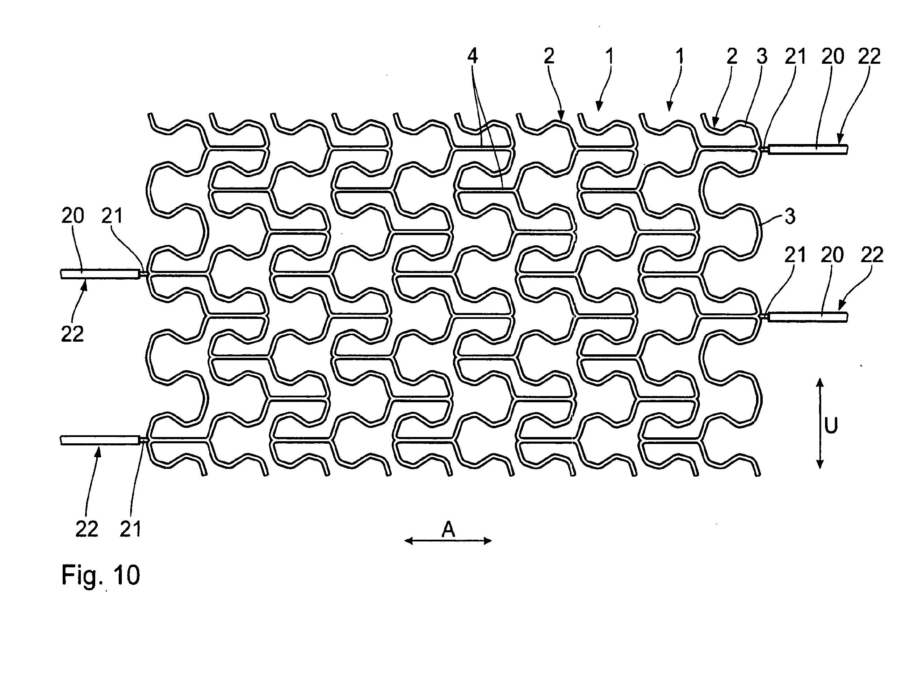

Anhand der Fig. 9 und 10 ist eine weitere Alternative für das Stabilisierungsmittel zur zusätzlichen radialen Fixierung des Stents dargestellt. Es handelt sich hierbei um Verlängerungsstreben 20, die im Wesentlichen in Axialrichtung A verlaufen und mit ihrem einen Ende über eine Sollbruchstelle 21 an der äußeren Strebe 2 an den Stent angebunden sind. Das freie Ende 22 der Verlängerungsstreben 20 ist jeweils an einem in Fig. 9 nur schematisch angedeuteten Ballonkatheter 23 in geeigneter Weise dauerhaft befestigt.

Die Verlängerungsstreben 21 wirken wiederum einer radialen Expansion insbesondere der Mäanderbögen 3 der äußeren Strebe 2 entgegen, wodurch ein Flaring unterbunden wird. Beim Dilatieren des Stents werden die Sollbruchstellen 21 aufgetrennt und der Stent damit von den Verlängerungsstreben 20 freigegeben.

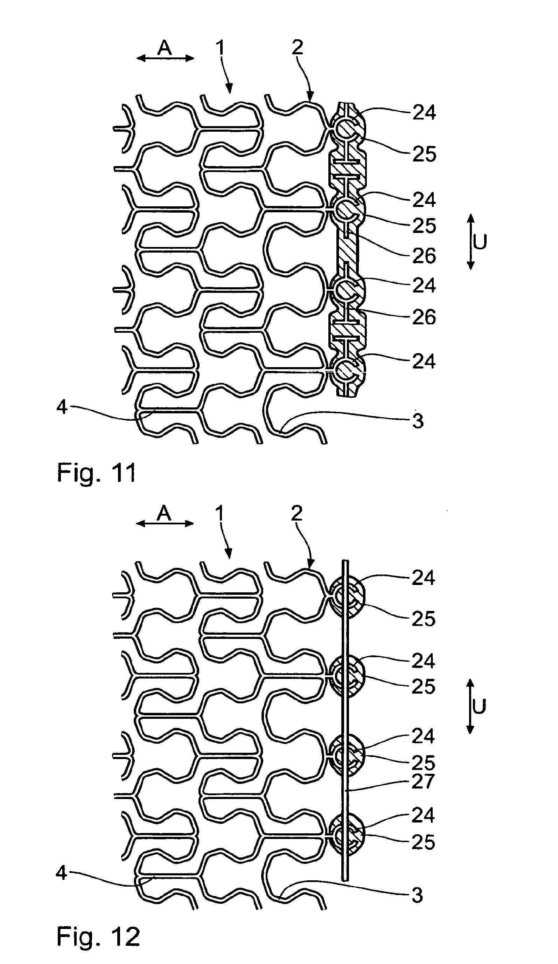

Die in den Fig. 11 und 12 gezeigten Ausführungsbeispiele für Stabilisierungsmittel gegen eine Flaring nützen die bei Stents oftmals vorhandenen Röntgenmarker-Fortsätze 24, die als ösenförmige Fortsätze an die Mäanderbögen 3 der äußeren Strebe 2 angeformt und mit einer thermoplastisch umformbaren Markerpolymerbeschichtung 25 versehen sind. Um diese bei Stents bekannten Elemente als Stabilisierungsmittel gegen ein Flaring zu verwenden, sind die Röntgenmarker-Fortsätze 24 mit ihrer Marker-Polymerbeschichtung 25 so ausgelegt, dass sie sich nach dem Krimpen des Stents berühren und durch eine thermische Behandlung der Marker-Polymerbeschichtung 25 zu einer umlaufenden Ring umformen. Die Ringbildung wird dabei durch in Peripherrichtung von den Röntgenmarker-Fortsätzen 24 abstehende Querverbindungselemente 26 unterstützt, die in Draufsicht entweder I- oder T-förmig gestaltet sein können.

Schließlich ist es - wie in Fig. 12 gezeigt ist - noch möglich, Stents mit polymerbeschichteten Röntgenmarker-Fortsätzen 24 so gegen ein Flaring auszurüsten, dass auf die Fortsätze 24 mit ihrer Beschichtung 25 ein umlaufender Ring beispielsweise aus Magnesium aufgeschoben ist, der durch ein Erwärmen und kurzes Aufschmelzen der Marker-Polymerbeschichtung 25 darin eingebettet und somit fixiert wird. Der Ring 27 kann aus steifem Material oder auch Draht bestehen, und auch nur auf einer Teil-Umfangslänge um den Stent gelegt sein.

## Patentansprüche

1. Stent, insbesondere aus einem Material mit einer geringen Festigkeit, mit einem eine Zylinderform umschreibenden, aus einer kontrahierten Ausgangsstellung in eine dilatierte Stützstellung radial expandierbaren Grundkörper umfassend
- eine Vielzahl in Umfangsrichtung (U) umlaufender, in Axialrichtung (A) aneinandergereihter Tragsegmente (1), die jeweils durch eine in ihrer kontrahierten Ausgangsstellung in ihrer Grobstruktur mäanderförmige Strebe (2) mit alternierend gegenläufigen, in die Stützstellung expandierbaren Mäanderbögen (3) aus biegsamen Material gebildet sind, und
- in axialparalleler Richtung (A) die Tragsegmente (1) vorzugsweise zwischen Zenitpunkten (5) zumindest eines Teils der Mäanderbögen (3) verbindende Axialverbinder (4), und
- - Röntgenmarker-Fortsätze (24) an den randseitigen Streben (2) zur Stabilisierung der Streben-Grobstruktur in ihrer kontrahierten Ausgangsstellung gegen ein radiales Aufweiten,
**dadurch gekennzeichnet, dass** die Röntgenmarker-Fortsätze (24)
- markerpolymerbeschichtet sind,
- durch die Markerpolymerbeschichtung (25) im kontrahierten Zustand ringförmig gekoppelt sind und
- bei einer radialen Expansion des Stents selbsttätig lösbar sind.

2. Stent nach Anspruch 1, **dadurch gekennzeichnet, dass** die Röntgenmarker-Fortsätze (24) mit radial gerichteten Querverbindungselementen (26) versehen sind.

3. Stent nach einem der Ansprüche 1 - 2 , **dadurch gekennzeichnet, dass** auf die Röntgenmarker-Fortsätze (24) im kontrahierten Stent-Zustand ein in die Markerpolymerbeschichtung (25) eingebetteter Ring (27), vorzugsweise Drahtring aufgezogen ist.

## Claims

1. A stent, in particular made of a material having low strength, comprising a base body that circumscribes a cylindrical shape and can be radially expanded from a contracted basic position into a dilated support position, comprising:
- a plurality of structural segments (1), which extend around the periphery in the circumferential direction (U) and are arranged next to each other in the axial direction (A) and which are each formed by a strut (2) that has a rough meander-shaped structure in the contracted basic position thereof and comprises alternately opposing meander curves (3), which can be expanded to assume the support position and are made of flexible material, and
- axial connectors (4), which in the axial-parallel direction (A) connect the structural segments (1), preferably between the peak points (5) of at least some of the meander curves (3), and
- X-ray marker appendices (24) at the struts (2) on the edge side so as to stabilize the rough strut structure in the contracted basic position thereof to prevent radial expansion,
**characterized in that** the X-ray marker appendices (24)
- are marker polymer-coated;
- are coupled in a ring-shaped manner in the contracted state by the marker polymer coating (25); and
- can be automatically detached during a radial expansion of the stent.

2. The stent according to claim 1, **characterized in that** the X-ray marker appendices (24) are provided with radially oriented transverse connection elements (26).

3. The stent according to either claim 1 or 2, **characterized in that** a ring (27), preferably a wire ring, which is embedded in the marker polymer coating (25), is pulled onto the X-ray marker appendices (24) in the contracted state of the stent.

## Revendications

1. Stent, en particulier constitué d'un matériau à faible résistance, avec un corps de base radialement expansible, d'une position de départ contractée vers une position d'appui dilatée, et décrivant une forme cylindrique, comprenant
- une multitude de segments de support (1) rangés les uns après les autres dans la direction axiale (A) et s'étendant sur la direction du pourtour (U), formés chacun par une branche (2) constituée d'un matériau souple et formant des méandres dans sa structure grossière dans la position de départ contractée, avec des courbes de méandres (3) s'inversant en alternance et expansibles dans la position d'appui, et
- des connecteurs axiaux (4) reliant les segments de support (1) parallèlement à la direction axiale (A), de préférence entre des sommets (5) d'au moins une partie des courbes de méandres (3), et
- des prolongements de marqueurs de radiographie (24) sur les branches (2) situées au bord, pour stabiliser la structure grossière de branches dans sa position de départ contractée, pour empêcher un élargissement radial, **caractérisé en ce que** les prolongements de marqueurs de radiographie (24)
- sont revêtus d'un polymère,
- sont accouplés en forme d'anneau par le revêtement polymère (25) dans l'état contracté, et
- se détachent automatiquement lors d'une expansion radiale du stent.

2. Stent selon la revendication 1, **caractérisé en ce que** les prolongements de marqueurs de radiographie (24) sont pourvus d'éléments de liaison transversale (26) orientés radialement.

3. Stent selon l'une des revendications 1 et 2, **caractérisé en ce que**, dans l'état contracté du stent, une bague (27), de préférence une bague en fil métallique enrobée dans le revêtement polymère (25), est tirée sur les prolongements de marqueurs de radiographie (24).
